# EUROPEAN PATENT APPLICATION

(11) **EP 4 693 202 A1**
(43) Date of publication of application: **11.02.2026**
(21) Application number: 23930818.2
(22) Date of filing: 09.11.2023
(51) Int. Cl.: G06T 15/08, A61B 6/03

(54) **IMAGE PROCESSING DEVICE, METHOD, AND PROGRAM**

(30) Priority: 29.03.2023 JP 2023053658
(71) Applicant: FUJIFILM Corporation, Tokyo Tokyo 106-8620 (JP)
(72) Inventor: OTAKE, Ryosuke, Tokyo 106-8620 (JP)
(74) Representative: Dehns Germany Partnerschaft mbB
(86) International application number: PCT/JP2023/040440
(87) International publication number: WO 2024/202189

(57) **Abstract**

A processor is configured to: acquire a three-dimensional organ image; derive correspondence points on the three-dimensional organ image, the correspondence points respectively corresponding to sampling points when volume rendering of a deformed three-dimensional organ image to which the three-dimensional organ image is deformed is to be performed; and use pixel values at the correspondence points on the three-dimensional organ image as pixel values at the sampling points to derive a rendering image which is to be acquired by performing the volume rendering of the deformed three-dimensional organ image.

## Description

### BACKGROUND OF THE INVENTION

### 1. Field of the Invention

The present disclosure relates to an image processing apparatus, method, and program.

### 2. Description of the Related Art

A three-dimensional image acquired by a computed tomography (CT) apparatus or a magnetic resonance imaging (MRI) apparatus, for example, is projected and displayed with a volume rendering method or a surface rendering method for use in diagnosis. In addition, a simulation of a surgical operation using a three-dimensional image is performed. For example, JP2014-176425A proposes a method of generating a solid mesh model of an organ such as a liver, accepting a deformation operation on the solid mesh model, and recreating and displaying the solid mesh model in accordance with the deformation operation.

### SUMMARY OF THE INVENTION

However, with the method described in JP2014-176425A, the solid mesh model is only deformed and displayed. Therefore, information of tissue inside an organ included in the three-dimensional image is lost. On the other hand, using a volume rendering method to two-dimensionally project a three-dimensional image and appropriately setting transparency make it possible to express information of tissue inside an organ. However, since an amount of data in a piece of volume data representing a three-dimensional image is large, deforming the three-dimensional image and then displaying the deformed image using the volume rendering method involves an issue of an increase in calculation cost.

In view of the circumstances described above, an object of the present disclosure is to make it possible to display information of internal tissue of a target structure in a deformed three-dimensional image at a low calculation cost.

An image processing apparatus according to the present disclosure includes at least one processor configured to:
acquire a three-dimensional organ image;
derive correspondence points on the three-dimensional organ image, the correspondence points respectively corresponding to sampling points when volume rendering of a deformed three-dimensional organ image to which the three-dimensional organ image is deformed is to be performed; and
use pixel values at the correspondence points on the three-dimensional organ image as pixel values at the sampling points to derive a rendering image which is to be acquired by performing the volume rendering of the deformed three-dimensional organ image.

Note that, in the image processing apparatus according to the present disclosure, the processor may be further configured to:
derive a solid mesh model representing the three-dimensional organ image;
deform the solid mesh model to derive a deformed solid mesh model corresponding to the deformed three-dimensional organ image; and
derive the correspondence points respectively corresponding to the sampling points based on displacement in the deformed solid mesh model from the solid mesh model before deformation.

In addition, in the image processing apparatus according to the present disclosure, the processor may be further configured to:
display the solid mesh model; and
accept deformation of the solid mesh model that is displayed to deform the solid mesh model.

In addition, in the image processing apparatus according to the present disclosure, the processor may be further configured to deform the solid mesh model in accordance with a boundary condition that is set for the solid mesh model.

In addition, in the image processing apparatus according to the present disclosure, the processor may be further configured to issue a warning when the boundary condition is abnormal.

In addition, in the image processing apparatus according to the present disclosure, the three-dimensional organ image may be a three-dimensional image of a liver.

In addition, in the image processing apparatus according to the present disclosure, the processor may be further configured to:
acquire a plurality of the three-dimensional organ images in different time phases;
align three-dimensional organ images in two time phases among the plurality of three-dimensional organ images to derive a displacement field representing displacement due to an elapse of time in respective pixels in the three-dimensional organ images in the two time phases; and
derive, in an intermediate time phase between the two time phases, the correspondence points on at least one of the three-dimensional organ images in the two time phases, the correspondence points respectively corresponding to sampling points when volume rendering of a deformed three-dimensional organ image to which the at least one of the three-dimensional organ images in the two time phases is deformed is to be performed, based on the displacement field.

In addition, in the image processing apparatus according to the present disclosure, the plurality of three-dimensional organ images may be three-dimensional organ images of a heart.

An image processing method according to the present disclosure includes causing a computer to:
acquire a three-dimensional organ image;
derive correspondence points on the three-dimensional organ image, the correspondence points respectively corresponding to sampling points when volume rendering of a deformed three-dimensional organ image to which the three-dimensional organ image is deformed is to be performed; and
use pixel values at the correspondence points on the three-dimensional organ image as pixel values at the sampling points to derive a rendering image which is to be acquired by performing the volume rendering of the deformed three-dimensional organ image.

An image processing program according to the present disclosure causes a computer to execute a process including:
acquiring a three-dimensional organ image;
deriving correspondence points on the three-dimensional organ image, the correspondence points respectively corresponding to sampling points when volume rendering of a deformed three-dimensional organ image to which the three-dimensional organ image is deformed is to be performed; and
using pixel values at the correspondence points on the three-dimensional organ image as pixel values at the sampling points to derive a rendering image which is to be acquired by performing the volume rendering of the deformed three-dimensional organ image.

According to the present disclosure, it is possible to display information of internal tissue of a target structure in a deformed three-dimensional image at a low calculation cost.

### BRIEF DESCRIPTION OF THE DRAWINGS

Fig. 1 is a diagram illustrating a schematic configuration of a diagnosis support system to which an image processing apparatus according to a first embodiment of the present disclosure is applied;
Fig. 2 is a diagram illustrating a hardware configuration of the image processing apparatus according to the first embodiment;
Fig. 3 is a functional configuration diagram of the image processing apparatus according to the first embodiment;
Fig. **4** is a functional configuration diagram of a derivation unit in the image processing apparatus according to the first embodiment;
Fig. 5 is a diagram illustrating an example of a solid mesh model of a liver;
Fig. 6 is a diagram illustrating the solid mesh models before and after deformation;
Fig. 7 is a diagram for explaining a warning;
Fig. 8 is a diagram for explaining volume rendering of a three-dimensional image;
Fig. 9 is a diagram for explaining volume rendering of the three-dimensional image that has been deformed;
Fig. 10 is a diagram for explaining derivation of correspondence points;
Fig. 11 is a diagram for explaining derivation of correspondence points;
Fig. 12 is a diagram illustrating a display screen for a rendering image;
Fig. 13 is a flowchart illustrating processing performed in the first embodiment;
Fig. 14 is a diagram schematically illustrating a four-dimensional image of a heart;
Fig. 15 is a functional configuration diagram of a derivation unit in an image processing apparatus according to a second embodiment;
Fig. 16 is a diagram for explaining derivation of a displacement field;
Fig. 17 is a diagram for explaining derivation of correspondence points in the second embodiment;
Fig. 18 is a diagram for explaining displaying of a four-dimensional image of a heart in the second embodiment; and
Fig. 19 is a flowchart illustrating processing performed in the second embodiment.

### DESCRIPTION OF THE PREFERRED EMBODIMENTS

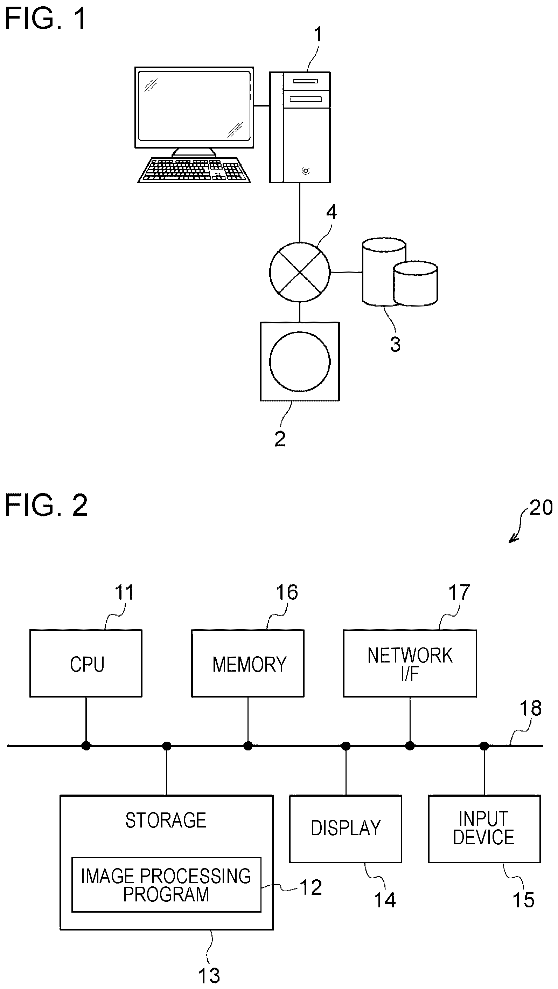

Embodiments of the present disclosure will be described below with reference to the accompanying drawings. A configuration of a medical information system to which an image processing apparatus according to a first embodiment is applied will first be described. Fig. 1 is a diagram illustrating a schematic configuration of the medical information system. In the medical information system illustrated in Fig. 1, a computer 1 including the image processing apparatus according to the present embodiment, an imaging device 2, and an image storage server 3 are coupled to each other in a communicable state via a network 4.

The computer 1 includes the image processing apparatus according to the present embodiment. In the computer 1, an image processing program according to the present embodiment is installed. The computer 1 may be a workstation or a personal computer that a doctor who performs a diagnosis directly operates, or may be a server computer coupled to the workstation or the personal computer via a network. The image processing program is stored in a storage device in a server computer coupled to a network or a network storage in an accessible state from outside, and is downloaded and installed, in accordance with a request, in the computer 1 that the doctor uses. The program may otherwise be recorded in a recording medium such as a digital versatile disc (DVD) or a compact disc read only memory (CD-ROM), and distributed and installed in the computer 1 from the recording medium.

The imaging device 2 is a device that captures an image of a portion of a subject, the portion serving as a target of diagnosis, and generates a three-dimensional image representing the portion, and is, specifically, a CT device, an MRI device, or a positron emission tomography (PET) device, for example. A three-dimensional image constituted by a plurality of tomographic images generated by the imaging device 2 is transmitted to and stored in the image storage server 3. Note that, in the present embodiment, the imaging device 2 is a CT device, and generates, for example, a CT image of a chest portion or an abdomen portion of a subject as the three-dimensional image.

The image storage server 3 is a computer that stores and manages various types of data, and includes a large-capacity external storage device and database management software. The image storage server 3 communicates with other devices via the network 4 in a wired or wireless manner to transmit and receive image data, for example. Specifically, the image storage server 3 acquires various types of data including image data of a CT image generated by the imaging device 2 via the network, and stores and manages the various types of data in a recording medium such as the large-capacity external storage device. Note that, a storage format for image data and communication between the devices via the network 4 are based on a protocol such as digital imaging and communication in medicine (DICOM).

Next, the image processing apparatus according to the first embodiment will be described. Fig. 2 is a diagram illustrating a hardware configuration of the image processing apparatus according to the present embodiment. As illustrated in Fig. 2, an image processing apparatus 20 includes a central processing unit (CPU) 11, a non-volatile storage 13, and a memory 16 serving as a temporary storage area. The image processing apparatus 20 further includes a display 14 such as a liquid crystal display, an input device 15 such as a keyboard and a mouse, and a network interface (I/F) 17 coupled to the network 4. The CPU 11, the storage 13, the display 14, the input device 15, the memory 16, and the network I/F 17 are coupled to a bus 18. Note that the CPU 11 is an example of a processor according to the present disclosure.

The storage 13 is implemented by a hard disk drive (HDD) or a solid state drive (SSD) and a flash memory, for example. An image processing program 12 is stored in the storage 13 serving as a storage medium. The CPU 11 reads out, from the storage 13, and develops, in the memory 16, the image processing program 12, and executes the image processing program 12 that has been developed.

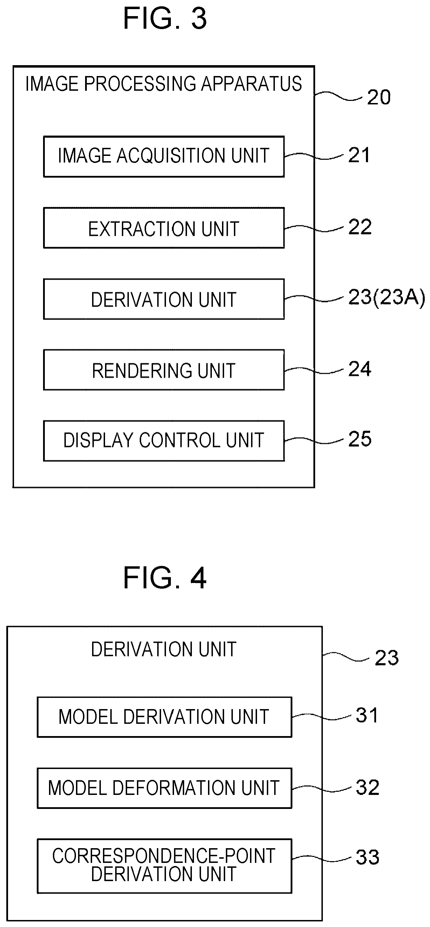

Next, a functional configuration of the image processing apparatus according to the first embodiment will be described. Fig. 3 is a diagram illustrating the functional configuration of the image processing apparatus according to the present embodiment. As illustrated in Fig. 3, the image processing apparatus 20 includes an image acquisition unit 21, an extraction unit 22, a derivation unit 23, a rendering unit 24, and a display control unit 25. When the CPU 11 executes the image processing program 12, the CPU 11 causes the image processing apparatus 20 to function as the image acquisition unit 21, the extraction unit 22, the derivation unit 23, the rendering unit 24, and the display control unit 25.

The image acquisition unit 21 acquires a three-dimensional image G0 serving as a target of processing from the image storage server 3 in response to an instruction from the input device 15 by the operator. In the present embodiment, the three-dimensional image G0 is a three-dimensional CT image constituted by a plurality of tomographic images including an abdomen portion of a human body.

The extraction unit 22 extracts a target organ from the three-dimensional image G0 to derive a three-dimensional organ image G1. In the first embodiment, the target organ is a liver, and the extraction unit 22 extracts a region of the liver from the three-dimensional image G0 to derive the three-dimensional organ image G1 of the liver. Specifically, the extraction unit 22 uses an extraction model that has undergone machine learning to extract the region of the liver from the three-dimensional image G0 to derive the three-dimensional organ image G1. Note that extraction of the three-dimensional organ image G1 is not limited to this case, and it is possible to use any method such as a method using template matching. In addition, the three-dimensional image G0 may be displayed on the display 14. An instruction, provided by an operator with respect to the displayed three-dimensional image G0, for extracting the region of the liver may be accepted. Thus, the region of the liver may be extracted from the three-dimensional image G0, and the three-dimensional organ image G1 may be derived.

Note that the extraction unit 22 may identify a main body of the liver, vessels (arteries, veins, and portal veins) in the liver, an inferior vena cava, and a gallbladder for derivation of the three-dimensional organ image G1. In this case, the main body of the liver, the vessels, the inferior vena cava, and the gallbladder each undergo segmentation for derivation of the three-dimensional organ image G1.

The derivation unit 23 derives correspondence points on the three-dimensional organ image G1, which respectively correspond to sampling points when volume rendering of a deformed three-dimensional organ image G2 to which the three-dimensional organ image G1 is deformed is to be performed. Fig. 4 is a diagram illustrating a functional configuration of the derivation unit 23 according to the first embodiment. In the first embodiment, as illustrated in Fig. 4, the derivation unit 23 includes a model derivation unit 31, a model deformation unit 32, and a correspondence-point derivation unit 33.

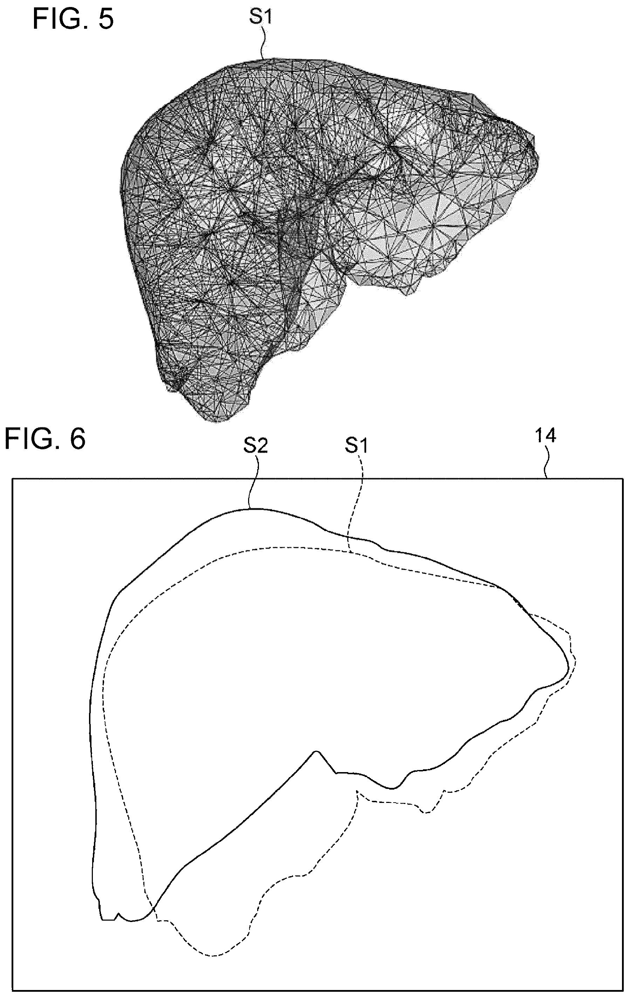

The model derivation unit 31 derives a solid mesh model of the three-dimensional organ image G1. Specifically, the model derivation unit 31 uses a well-known method such as a 3D Delauney division method to derive a solid mesh model serving as an aggregate of solid meshes (tetrahedron blocks) used in a finite element method in which physical properties, for example, for each portion forming the liver are reflected. Note that a modulus of elasticity is set to each of the solid meshes to express the physical properties of the liver in the solid mesh model. Fig. 5 is a diagram illustrating an example of a solid mesh model S1 of the liver, which is derived by the model derivation unit 31.

The model deformation unit 32 causes the display 14 to display the solid mesh model S1 of the liver, which is derived by the model derivation unit 31. Then, the model deformation unit 32 accepts an instruction, provided by the operator via the input device 15, for deforming the solid mesh model S1, and deforms the solid mesh model S1.

In a surgical operation for a liver serving as a target, a surgical operator may lift and incise the liver during the surgical operation. When the liver is lifted and incised, the liver deforms. The vessels run inside the liver. When the liver is to be incised, in particular, it may be desired, in preoperative planning, to simulate how the vessels run inside the deformed liver.

When such a simulation is to be performed, deforming the three-dimensional organ image G1 as assumed during a surgical operation and performing volume rendering of the deformed three-dimensional organ image make it possible to check a state of internal tissue of the deformed liver. However, since an amount of data in volume data representing the three-dimensional organ image G1 is large, deforming the three-dimensional organ image G1 and performing volume rendering lead to an increase in calculation cost.

Therefore, in the first embodiment, the solid mesh model S1 of the liver is derived from the three-dimensional organ image G1, and deformation with respect to the solid mesh model S1 is accepted.

Under an assumed case where the operator lifts and incises the liver, the solid mesh model S1 is deformed. For this purpose, the model deformation unit 32 stores, for vertices of solid meshes forming a solid mesh model S2 after deformation, amounts of displacement with respect to respective vertices of solid meshes forming the solid mesh model S1. An amount of displacement is set as described below.

For example, when, in preoperative planning, a liver is deformed and volume rendering in which a result of the deformation is reflected is to be performed, it is possible to use a method of acquiring a result of analysis using a static-analysis-based finite element method, based on boundary conditions including a constraint condition and a load condition input by the operator. Note that, as the constraint condition in the boundary conditions, that is, a position where the liver is fixed, it is possible to use a portion where the vena cava and the main body of the liver are coupled to each other. Accordingly, when the operator uses the input device 15 to deform the solid mesh model S1 displayed on the display 14, the solid mesh model S1 is deformed into a shape designated by the operator in a state where the portion where the vena cava and the main body of the liver are coupled to each other is fixed. Note that the input device 15 is used to input the boundary conditions to the image processing apparatus 20 before a deformation operation is performed.

Fig. 6 is a diagram illustrating the solid mesh models before and after deformation. Fig. 6 illustrates a state where the solid mesh model S2 after deformation is superimposed on the solid mesh model S1 before deformation. In Fig. 6, displaying of the solid meshes in the solid mesh models S1 and S2 is omitted for purposes of explanation. Note that only the solid mesh model S2 after deformation may be displayed on the display 14.

Then, the model deformation unit 32 stores amounts of displacement at the vertices on the solid mesh model S2 after deformation respectively with respect to the corresponding vertices on the solid mesh model S1 before deformation in the storage 13.

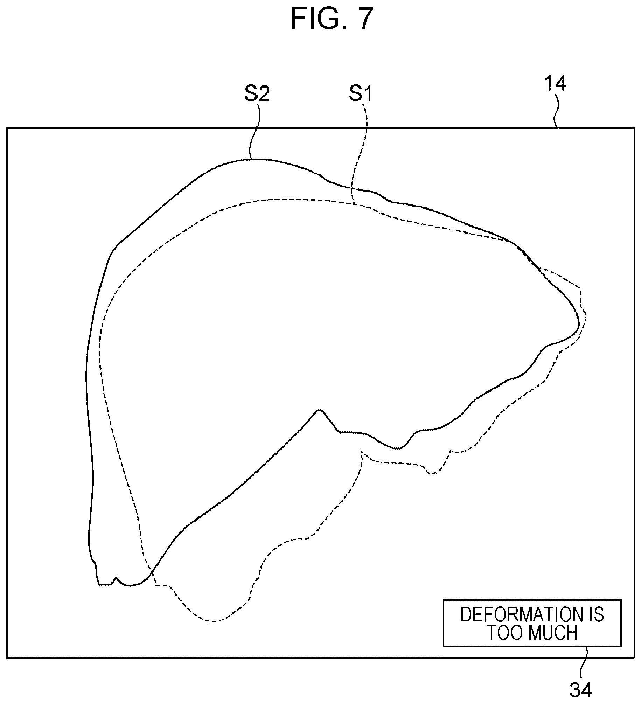

Note that, when the solid mesh model S1 is to be deformed and boundary conditions are to be set, amounts of deformation and boundary conditions, which are not actually feasible, (for example, load and constraint conditions that are not actually feasible) may be set. In such a case, the model deformation unit 32 may preferably calculate maximum stress to be generated in an organ due to the deformation and the boundary conditions, determine that there is an abnormality in the boundary conditions when the maximum stress exceeds a threshold value Th, and issue a warning. For example, as illustrated in Fig. 7, the warning may be provided in a form of text 34 such as "Deformation is too much" displayed on the display 14, or may be provided in a form of a notification sound.

The correspondence-point derivation unit 33 derives correspondence points on the three-dimensional organ image G1, which respectively correspond to sampling points when volume rendering of the deformed three-dimensional organ image G2 to which the three-dimensional organ image G1 is deformed is to be performed, based on the respective amounts of displacement between the solid mesh models before and after deformation, which are derived by the model deformation unit 32.

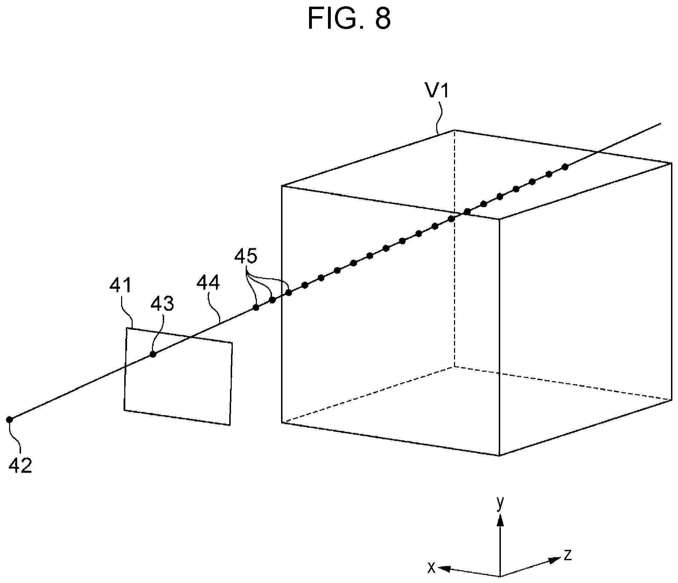

Volume rendering of a three-dimensional image will now be described. Fig. 8 is a diagram for explaining volume rendering of a three-dimensional image. When volume rendering of a three-dimensional image V1 is to be performed, a projection surface 41 corresponding to a screen of the display 14 is set, and ray-casting is performed to perform rendering of the three-dimensional image V1 on the projection surface 41.

The projection surface 41 is, for example, a virtual plane defined at a resolution corresponding to a resolution of the screen of the display 14. As the ray-casting is performed on the three-dimensional image V1, a virtual light ray passing through each of pixels on the projection surface 41 from a viewpoint 42 passes through the three-dimensional image V1 and is projected onto the projection surface 41. Note that, in Fig. 8, only a light ray 44 passing through a pixel 43 on the projection surface 41 is illustrated.

A position of the viewpoint 42 with respect to the three-dimensional image V1 is changed in accordance with, for example, an instruction accepted by the input device 15, whereby a rendering image when the three-dimensional image V1 is observed from each of various directions is projected onto the projection surface 41.

When volume rendering is to be performed, each of pixel values of the three-dimensional image V1, that is, each of pieces of voxel data, is converted into an RGBα value indicating a degree of transparency α in addition to each of colors (that is, RGB). In the conversion, a correspondence table in which the RGBα value is defined in accordance with a value of a piece of CT data is used.

When ray-casting is to be performed, data (hereinafter also referred to as "accumulation data") acquired by accumulating RGBα values at a plurality of sampling points 45 (for example, points defined at one-voxel intervals) on the light ray 44 is projected onto the projection surface 41, as illustrated in Fig. 8. The accumulation data that has been projected represents the pixel value of the pixel 43. Note that, when a sampling point is positioned between voxels in the three-dimensional image V1, an RGBα value at the sampling point 45 is acquired through interpolation calculation using the RGBα values of the voxels around the sampling point 45. By performing such derivation of the accumulation data for all the pixels on the projection surface 41, a rendering image is generated through volume rendering.

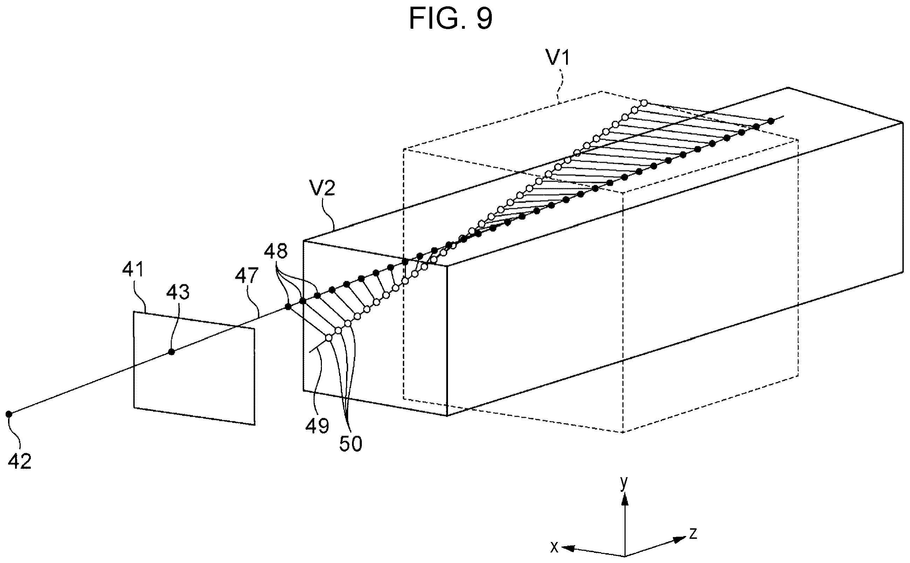

As illustrated in Fig. 9, volume rendering on a three-dimensional image V2, acquired by deforming the three-dimensional image V1 illustrated in Fig. 8 to shrink in x and y directions and expand in a z direction, will now be described. As illustrated in Fig. 9, when ray-casting is to be performed for a light ray 47 that passes through the three-dimensional image V2 and passes through the pixel 43 on the projection surface 41 from the viewpoint 42, it is possible to acquire accumulation data of RGBα values respectively at a plurality of sampling points 48 on the light ray 47 as in Fig. 8, to acquire a pixel value of the pixel 43 on the projection surface 41.

On the other hand, since the three-dimensional image V2 has been acquired by deforming the three-dimensional image V1, it is possible to associate voxels representing an identical structure in the three-dimensional image V1 and the three-dimensional image V2 with each other in a one-to-one manner. For example, it is possible to associate each of the sampling points 48 on the light ray 47 for the three-dimensional image V2 in a one-to-one manner with a corresponding one of points 50 on a light ray 49 in the three-dimensional image V1. Therefore, when it is possible to identify points on the three-dimensional image V1, which respectively correspond to the sampling points 48 on the three-dimensional image V2, that is, when it is possible to identify correspondence points, it is possible to acquire RGBα values at the sampling points 48 on the three-dimensional image V2 without deforming the three-dimensional image V1 to derive a three-dimensional image V2. As a result, it is possible to generate a rendering image for the three-dimensional image V2 without deforming the three-dimensional image V1 to derive a three-dimensional image V2.

In the first embodiment, the correspondence-point derivation unit 33 derives correspondence points on the three-dimensional organ image G1, which respectively correspond to sampling points when volume rendering of the deformed three-dimensional organ image G2 to which the three-dimensional organ image G1 is deformed is to be performed, based on amounts of displacement at vertices of solid meshes forming the solid mesh models S1 and S2 before and after deformation, which are derived by the model deformation unit 32.

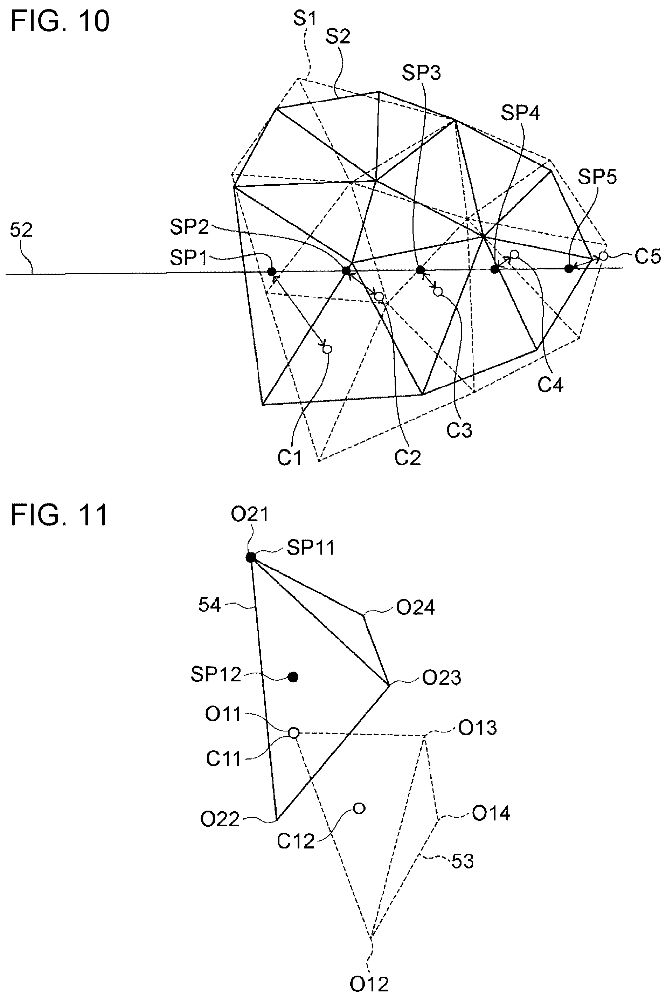

Fig. 10 is a diagram for explaining derivation of correspondence points. Note that, in Fig. 10, for simplification of description, the solid mesh models S1 and S2 are illustrated in a two-dimensional manner. That is, the solid mesh models S1 and S2 are respectively represented by aggregates of triangular polygons. In addition, in Fig. 10, the solid mesh model S1 before deformation is indicated by a broken line, and the solid mesh model S2 after deformation is indicated by a solid line.

The solid mesh model S2 after deformation corresponds to the deformed three-dimensional organ image G2 to which the three-dimensional organ image G1 before deformation is deformed. Fig. 10 illustrates a state where five sampling points SP1 to SP5 are set, with respect to the solid mesh model S2, at equal intervals on a light ray 52 when volume rendering of the deformed three-dimensional organ image G2 is to be performed.

When the solid mesh model S1 is deformed into the solid mesh model S2 as described above, positions of vertices of solid meshes in the solid mesh model S1 are changed in accordance with predetermined boundary conditions, and the solid mesh model S2 is derived. Therefore, it is possible to associate the vertices of the solid meshes in the solid mesh model S1 and the vertices of the solid meshes in the solid mesh model S2 with each other in a one-to-one manner.

As illustrated in Fig. 11, a solid mesh 53 included in the solid mesh model S1 is assumed to be deformed into a solid mesh 54. In Fig. 11, four vertices O11 to 014 of the solid mesh 53 correspond to vertices O21 to O24 of the solid mesh 54, respectively. In this case, when a sampling point SP11 coincides with the vertex O21 of the solid mesh 54, for example, the vertex O11 of the solid mesh 53 is regarded as a correspondence point C11 corresponding to the sampling point SP11.

When a sampling point SP12 is positioned within the solid mesh 54, as illustrated in Fig. 11, on the other hand, the correspondence-point derivation unit 33 derives relative coordinate positions of the sampling point SP12 with respect to the four vertices O21, O22, O23, and O24 of the solid mesh 54, and derives distances L1 to L4 from the four vertices O21, O22, O23, and O24 to the sampling point SP12, respectively. Then, the correspondence-point derivation unit 33 derives, as a correspondence point C12, a coordinate position on the three-dimensional organ image G1 before deformation, which is positioned within the solid mesh 53 and at which the distances from the four vertices O11, 012, 013, and O14 correspond to L1:L2:L3:L4, respectively, in the solid mesh 53 before deformation.

As illustrated in Fig. 10, the correspondence-point derivation unit 33 derives correspondence points C1 to C5 in the solid mesh model S1 before deformation respectively for the sampling points SP1 to SP5 in the solid mesh model S2 after deformation.

The rendering unit 24 uses, as RGBα values at the sampling points on the deformed three-dimensional organ image G2, RGBα values at the correspondence points on the three-dimensional organ image G1, which have been derived by the correspondence-point derivation unit 33 in the derivation unit 23, to perform ray-casting, and derives a rendering image R2 after deformation. For example, for the sampling points SP1 to SP5 illustrated in Fig. 10, the RGBα values respectively at the correspondence points C1 to C5 in the solid mesh model S1 are accumulated to derive a pixel value of a pixel corresponding to those on the light ray 52. The rendering unit 24 performs this operation for all images on the projection surface to derive the rendering image R2 after deformation.

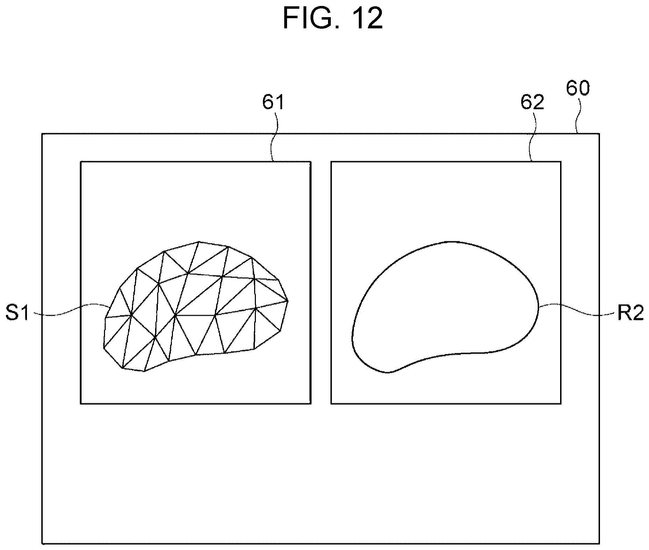

The display control unit 25 causes the display 14 to display the rendering image. Fig. 12 is a diagram illustrating a display screen for a rendering image. As illustrated in Fig. 12, a display screen 60 has a first display region 61 for displaying the solid mesh model and a second display region 62 for displaying the rendering image R2 after deformation. Accordingly, it is possible to allow the operator, by deforming the solid mesh model displayed in the first display region 61 to display, in the second display region 62, the rendering R2 image which is derived as volume rendering of the three-dimensional organ image G2 after deformation is performed.

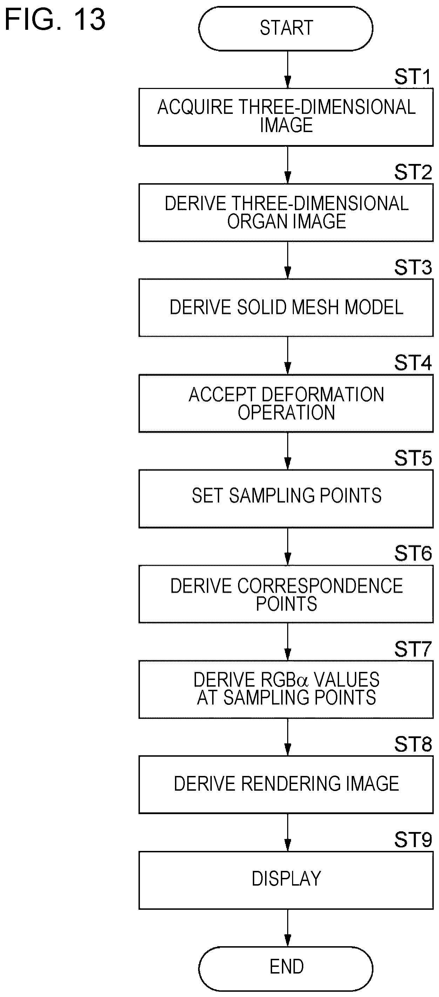

Next, processing to be performed in the first embodiment will now be described. Fig. 13 is a flowchart illustrating the processing to be performed in the first embodiment. The image acquisition unit 21 first acquires a three-dimensional image G0 serving as a target of processing (step ST1). The extraction unit 22 extracts a liver serving as a target organ from the three-dimensional image G0 to derive a three-dimensional organ image G1 (step ST2).

Next, the model derivation unit 31 in the derivation unit 23 derives a solid mesh model S1 of the three-dimensional organ image G1 (step ST3). A deformation operation for the solid mesh model S1 by the operator is subsequently accepted (step ST4). Accordingly, the model deformation unit 32 deforms the solid mesh model S1 to derive a solid mesh model S2 that has been deformed.

Then, the correspondence-point derivation unit 33 sets sampling points respectively for pixels on the projection surface 41 when volume rendering of the solid mesh model S2 that has been deformed is to be performed (step ST5). Furthermore, the correspondence-point derivation unit 33 derives correspondence points on the three-dimensional organ image G1 before deformation, which respectively correspond to the sampling points (step ST6).

The rendering unit 24 subsequently derives RGBα values at the sampling points based on RGBα values at the correspondence points (step ST7). Furthermore, the rendering unit 24 accumulates the RGBα values at the sampling points to derive respective pixel values of pixels on the projection surface 41 to derive a rendering image (step ST8). Then, the display control unit 25 causes the display 14 to display the rendering image (step ST9). The processing then ends.

In the first embodiment, as described above, correspondence points on a three-dimensional organ image, which respectively correspond to sampling points when volume rendering of a deformed three-dimensional organ image G2 to which a three-dimensional organ image G1 is deformed is to be performed, are derived, and pixel values at the correspondence points on the three-dimensional organ image G1 are used as pixel values at the sampling points to derive a rendering image for the deformed three-dimensional organ image G2. Therefore, it is possible to derive a rendering image acquired by performing volume rendering of the deformed three-dimensional organ image G2 without deforming the three-dimensional organ image G1 to derive a deformed three-dimensional organ image G2. In a rendering image derived through volume rendering, information of not only a surface of an organ but also internal tissue of the organ is rendered. Therefore, according to the first embodiment, it is possible to display information of internal tissue of an organ included in a deformed three-dimensional image at a low calculation cost.

In addition, in the first embodiment, a solid mesh model S1 representing the three-dimensional organ image G1 is derived, the solid mesh model S1 is deformed, and the correspondence points respectively corresponding to the sampling points are derived based on displacement in the solid mesh model S2 that has been deformed from the solid mesh model S1 before deformation. Therefore, it is possible to derive a rendering image of an organ deformed as desired by the operator.

In addition, setting boundary conditions for a solid mesh model makes it possible to set, in an actual surgical operation, for example, a point that does not move even when an organ is deformed and a point that moves when the organ is deformed. Accordingly, it is possible to deform the organ in a state similar to that in an actual surgical operation, and thus it is possible to accurately perform a simulation of the surgical operation.

Next, a second embodiment of the present disclosure will be described. Note that a functional configuration of an image processing apparatus according to the second embodiment is identical to that of the image processing apparatus according to the first embodiment illustrated in Fig. 3, and its detailed description will be omitted.

In the first embodiment described above, a three-dimensional organ image of a liver is used, and volume rendering of a deformed three-dimensional organ image G2 for the liver when deformed is performed to derive a rendering image. The second embodiment differs from the first embodiment in that a three-dimensional image included in a four-dimensional image of a heart is used as a three-dimensional organ image to perform volume rendering of a deformed three-dimensional organ image.

A four-dimensional image is an image that makes it possible to reproduce a movement of a certain organ as a motion picture by continuously performing imaging at short time intervals, acquiring three-dimensional images of the certain organ in an identical photographic subject, and displaying in a time series order rendering images derived by performing volume rendering of the three-dimensional images that have been acquired. Therefore, a four-dimensional image of a heart is an image that makes it possible to represent a movement due to beating of the heart by performing imaging at short time intervals, acquiring a plurality of three-dimensional organ images of the heart, acquiring rendering images of the heart by performing volume rendering of the plurality of three-dimensional organ images that have been acquired, and displaying in a time series order the rendering images that have been acquired.

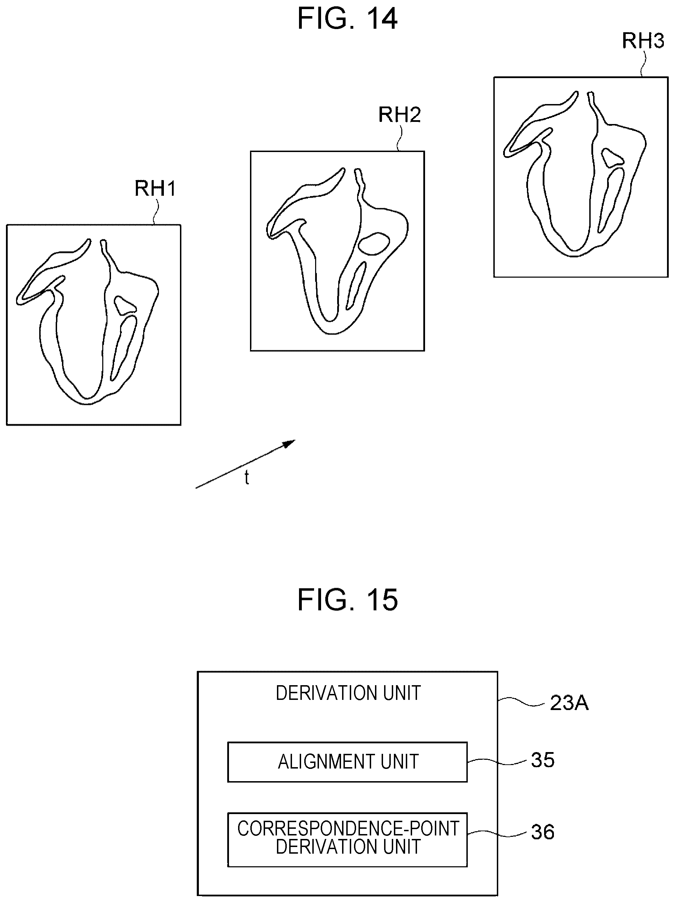

Fig. 14 is a diagram schematically illustrating a four-dimensional image of a heart. As illustrated in Fig. 14, a four-dimensional image of a heart is an image representing a movement of the heart by displaying three-dimensional images of the heart in a time series order. Note that, in Fig. 14, the three-dimensional images are displayed in a time series order by arranging rendering images RH1 to RH3 having undergone volume rendering along a time axis t. The rendering image RH1 and the rendering image RH3 are images of the heart in a diastolic phase, and the rendering image RH2 is an image of the heart in a systolic phase.

Newly deriving, between two three-dimensional organ images in different time phases, a deformed three-dimensional organ image that interpolates a gap between the two time phases by deforming the three-dimensional organ image in one of the time phases makes it possible to more smoothly express, when the three-dimensional organ images and deformed three-dimensional organ images are displayed in a time series order, a movement of the organ, that is, the heart. However, deforming a three-dimensional organ image increases a calculation cost. In the second embodiment, a new rendering image that interpolates a gap between two time phases is derived without deforming a three-dimensional organ image.

In the second embodiment, the image acquisition unit 21 acquires a four-dimensional image of a heart from the image storage server 3. The extraction unit 22 extracts the heart from each of a plurality of three-dimensional images in different time phases, which are included in the four-dimensional image of the heart, and derives a three-dimensional organ image of the heart.

Fig. 15 is a diagram illustrating a functional configuration of a derivation unit according to the second embodiment. As illustrated in Fig. 15, a derivation unit 23A according to the second embodiment includes an alignment unit 35 and a correspondence-point derivation unit 36.

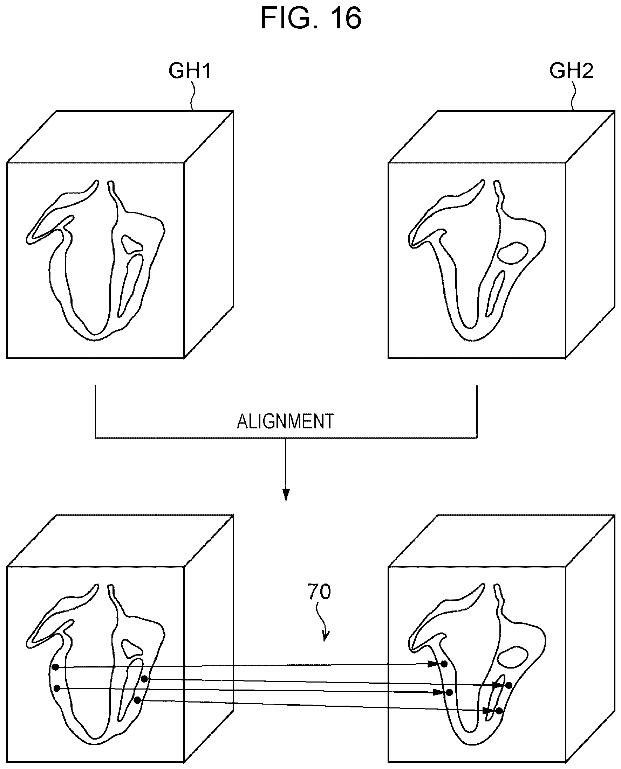

The alignment unit 35 aligns two three-dimensional organ images in different time phases to derive a displacement field representing displacement of pixels corresponding to each other in the two three-dimensional organ images. Fig. 16 is a diagram for explaining derivation of a displacement field. Fig. 16 explains derivation of a displacement field between a three-dimensional organ image GH1 representing the heart in the diastolic phase and a three-dimensional organ image GH2 representing the heart in the systolic phase. In the second embodiment, the alignment unit 35 performs non-rigid aligning to perform aligning between the three-dimensional organ image GH1 and the three-dimensional organ image GH2, and derives a displacement field 70 representing a correspondence relationship between the three-dimensional organ image GH1 and the three-dimensional organ image GH2 for the pixels corresponding to each other in the three-dimensional organ image GH1 and the three-dimensional organ image GH2. The displacement field 70 is constituted by vectors to the pixels in the three-dimensional organ image GH2, which respectively correspond to the pixels in the three-dimensional organ image GH1. These vectors are referred to as displacement vectors. Note that, although Fig. 16 illustrates the displacement field 70 constituted by only the displacement vectors for some of the pixels, the displacement field 70 is acquired by acquiring displacement vectors based on alignment for all the pixels in the three-dimensional organ image GH1 and the three-dimensional organ image GH2.

When a three-dimensional organ image in a time phase between the time phase of the three-dimensional organ image GH1 and the time phase of the three-dimensional organ image GH2 is to be derived as a deformed organ image, the correspondence-point derivation unit 36 derives correspondence points, for sampling points when volume rendering of a deformed three-dimensional organ image is to be performed, on the three-dimensional organ image GH1 or three-dimensional organ image GH2 in one of the two time phases, based on the displacement field 70 derived by the alignment unit 35. Note that the deformed three-dimensional organ image is a new three-dimensional organ image for use in interpolation between the two time phases.

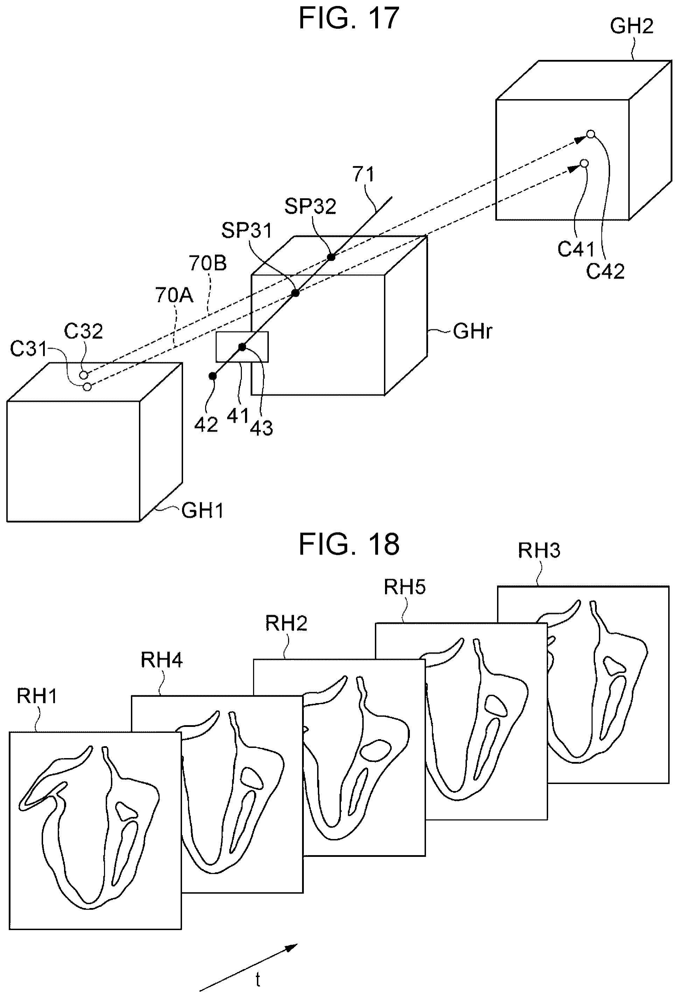

Fig. 17 is a diagram for explaining derivation of correspondence points in the second embodiment. Note that Fig. 17 illustrates derivation of correspondence points for sampling points on a deformed three-dimensional organ image GHr in an intermediate time phase between the two time phases of the three-dimensional organ image GH1 and the three-dimensional organ image GH2. The correspondence-point derivation unit 36 sets a virtual deformed three-dimensional organ image GHr in which a pixel position is positioned at a point that equally divides a displacement vector included in the displacement field 70 between the three-dimensional organ image GH1 and the three-dimensional organ image GH2 in the two time phases into two pieces.

As illustrated in Fig. 17, when ray-casting is to be performed for a light ray 71 passing through the pixel 43 on the projection surface 41 from the viewpoint 42 in the deformed three-dimensional organ image GHr, the correspondence-point derivation unit 36 derives correspondence points on the three-dimensional organ image GH1 or the three-dimensional organ image GH2, which respectively correspond to sampling points on the light ray 71. Note that, although Fig. 17 only illustrates two sampling points SP31 and SP32, more sampling points are actually set on the light ray 71.

When a sampling point is positioned in a voxel on the deformed three-dimensional organ image GHr, the correspondence-point derivation unit 36 derives a voxel on the three-dimensional organ image GH1 or the three-dimensional organ image GH2, which corresponds to the sampling point, as a correspondence point, based on a displacement vector included in the displacement field 70, which passes through the voxel. When a sampling point is positioned between voxels on the deformed three-dimensional organ image GHr, a correspondence point, which corresponds to a sampling point, on the three-dimensional organ image GH1 or the three-dimensional organ image GH2 is derived based on displacement vectors respectively passing through a plurality of voxels around the sampling point and distances respectively between the sampling point and the plurality of voxels around the sampling point. In this case, the correspondence point may be positioned between voxels on the three-dimensional organ image GH1 or the three-dimensional organ image GH2.

Note that, in Fig. 17, for the sampling point SP31 on the light ray 71, a correspondence point C31 on the three-dimensional organ image GH1 and a correspondence point C41 on the three-dimensional organ image GH2 are derived based on a displacement vector 70A. In addition, for the sampling point SP32 on the light ray 71, a correspondence point C32 on the three-dimensional organ image GH1 and a correspondence point C42 on the three-dimensional organ image GH2 are derived based on a displacement vector 70B.

The rendering unit 24 uses, as RGBα values at sampling points on the deformed three-dimensional organ image GHr, RGBα values at the correspondence points on the three-dimensional organ image GH1 or the three-dimensional organ image GH2, which have been derived by the derivation unit 23A, to perform ray-casting, and derives a rendering image RH2 in a time phase between the two time phases. For the sampling point SP31 illustrated in Fig. 17, for example, an RGBα value at the correspondence point C31 on the three-dimensional organ image GH1 or the correspondence point C41 on the three-dimensional organ image GH2 is used. In addition, for the sampling point SP32 illustrated in Fig. 17, an RGBα value at the correspondence point C32 on the three-dimensional organ image GH1 or the correspondence point C42 on the three-dimensional organ image GH2 is used.

The display control unit 25 uses the rendering image derived by the derivation unit 23A in addition to the rendering image for the three-dimensional images included in the four-dimensional image of the heart, which are acquired by the image acquisition unit 21, to cause the four-dimensional image of the heart to be displayed. Fig. 18 is a diagram for explaining displaying of a four-dimensional image of a heart in the second embodiment. In a conventional four-dimensional image of a heart, as illustrated in Fig. 14 described above, the rendering images RH1 to RH3 based on three-dimensional images of a heart, which are acquired through imaging using a CT apparatus, are displayed in a time series order. In the second embodiment, as illustrated in Fig. 18, on the other hand, in addition to the rendering images based on the three-dimensional images acquired through imaging, new rendering images RH4 and RH5 based on a deformed three-dimensional organ image in a time phase between two time phases are displayed between the rendering image RH1 and the rendering image RH2 and between the rendering image RH2 and the rendering image RH3, respectively.

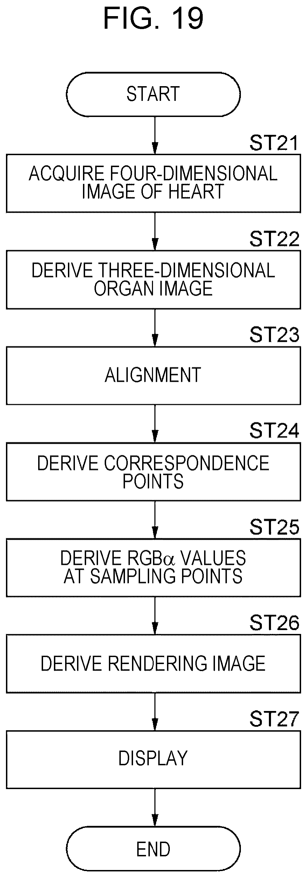

Next, processing to be performed in the second embodiment will be described. Fig. 19 is a flowchart illustrating the processing to be performed in the second embodiment. The image acquisition unit 21 first acquires a four-dimensional image of a heart, which serves as a target of processing (step ST21). The extraction unit 22 extracts the heart serving as a target organ from each of three-dimensional images included in the four-dimensional image of the heart to derive three-dimensional organ images GH1 to GH3 (step ST22).

Next, the alignment unit 35 in the derivation unit 23A aligns two of the three-dimensional organ images in different time phases to derive a displacement field representing displacement of corresponding pixels on the two three-dimensional organ images (alignment; step ST23). Next, the correspondence-point derivation unit 36 derives correspondence points, for sampling points when volume rendering of the deformed three-dimensional organ image is to be performed, on the three-dimensional organ image GH1 or the three-dimensional organ image GH2 in one of the two time phases, based on the displacement field derived by the alignment unit 35 (step ST24).

The rendering unit 24 subsequently derives RGBα values at the sampling points based on RGBα values at the correspondence points (step ST25), accumulates the RGBα values at the sampling points, derives respective pixel values at the pixels on the projection surface 41, and derives a rendering image (step ST26). Then, the display control unit 25 causes the display 14 to display the rendering image of the four-dimensional image of the heart including the newly derived rendering image (step ST27). The processing then ends.

In the second embodiment, as described above, correspondence points on a three-dimensional organ image, which respectively correspond to sampling points when volume rendering of a deformed three-dimensional organ image in a time phase between two three-dimensional organ images in different time phases are derived, and pixel values at the correspondence points on the three-dimensional organ image are used as pixel values at the sampling points to derive a rendering image of the deformed three-dimensional organ image G2. Therefore, when a rendering image for use in interpolation in time phase between three-dimensional images included in a four-dimensional image of a heart is to be derived, it is possible to derive a rendering image acquired by performing volume rendering of the deformed three-dimensional organ image G2 without deforming the three-dimensional organ image G1 to derive the deformed three-dimensional organ image G2. In a rendering image derived through volume rendering, information of not only a surface of an organ but also internal tissue of the organ is rendered. Therefore, according to the second embodiment, it is possible to display a four-dimensional image of a heart, which changes smoothly, including information of its inside, at a low calculation cost.

Note that, although, in the second embodiment described above, a deformed three-dimensional organ image is virtually set at a point that equally divides a displacement vector included in a displacement field into two pieces, the present disclosure is not limited to this case. Deformed three-dimensional organ images may be virtually set at points that equally divide a displacement vector into three or four pieces. Accordingly, it is possible to more finely divide a time phase and display a four-dimensional image of a heart that more smoothly represents a movement of the heart.

In addition, in the second embodiment described above, a four-dimensional image of lungs may be used instead of a four-dimensional image of a heart. In this case, when a rendering image is to be displayed to represent a three-dimensional movement of the lungs due to respiration, it is possible to achieve a smooth movement. In addition, a four-dimensional image of a circulatory system including both the heart and the lungs may be used. In this case, a rendering image is four-dimensionally displayed to smoothly represent both a three-dimensional movement of the heart due to beating and a three-dimensional movement of the lungs due to respiration.

In addition, although a target organ is a liver in the first embodiment described above, the target organ is not limited to the liver. It is possible to set, as a target, any organ such as a pancreas, a stomach, lungs, or a spleen, which may deform during a surgical operation.

In addition, although, in the embodiments described above, a CT image is used as a three-dimensional image, the present disclosure is not limited to this case. A three-dimensional image such as an MRI image may be used.

In addition, in the embodiments described above, for example, the image processing apparatus 20 may use various types of processors, as described below, as a hardware structure of processing units that execute various types of processing such as the image acquisition unit 21, the extraction unit 22, the derivation units 23 and 23A, the rendering unit 24, the display control unit 25, the model derivation unit 31, the model deformation unit 32, the correspondence-point derivation unit 33, the alignment unit 35, and the correspondence-point derivation unit 36. As described above, the various types of processors, as described above, include, in addition to a CPU that is a general-purpose processor that executes pieces of software (programs) to function as the various processing units, for example, a programmable logic device (PLD) that is a processor whose circuit configuration can be changed after manufactured, such as a field programmable gate array (FPGA), and a dedicated electric circuit that is a processor having a circuit configuration designed exclusively for executing certain processing, such as an application specific integrated circuit (ASIC).

One processing unit may be configured by one of the various types of processors, or may be configured by a combination of two or more of the processors of the identical type or different types (for example, a combination of a plurality of FPGAs or a combination of a CPU and an FPGA). In addition, a plurality of processing units may be configured by one processor.

As an example when a plurality of processing units are configured by one processor, there is firstly a form where, as represented by computers such as a client and a server, one or more CPUs and pieces of software are combined with each other to configure one processor, and the processor functions as a plurality of processing units. There is secondly a form where, as represented by a system on chip (SoC), for example, a processor in which one integrated circuit (IC) chip achieves functions of a whole system including a plurality of processing units is used. In this way, one or more of the various types of processors is or are used to configure the various processing units as described above as a hardware structure.

Furthermore, it is possible to use, as a hardware structure of the various types of processors, more specifically, an electric circuit (circuitry) in which circuit elements such as semiconductor elements are combined with each other.

Appendices of the present disclosure will now be described herein.

### (Appendix 1)

An image processing apparatus including at least one processor configured to:
acquire a three-dimensional organ image;
derive correspondence points on the three-dimensional organ image, the correspondence points respectively corresponding to sampling points when volume rendering of a deformed three-dimensional organ image to which the three-dimensional organ image is deformed is to be performed; and
use pixel values at the correspondence points on the three-dimensional organ image as pixel values at the sampling points to derive a rendering image which is to be acquired by performing the volume rendering of the deformed three-dimensional organ image.

### (Appendix 2)

The image processing apparatus according to Appendix 1, in which the processor is further configured to:
derive a solid mesh model representing the three-dimensional organ image;
deform the solid mesh model to derive a deformed solid mesh model corresponding to the deformed three-dimensional organ image; and
derive the correspondence points respectively corresponding to the sampling points based on displacement in the deformed solid mesh model from the solid mesh model before deformation.

### (Appendix 3)

The image processing apparatus according to Appendix 2, in which the processor is further configured to:
display the solid mesh model; and
accept deformation of the solid mesh model that is displayed to deform the solid mesh model.

### (Appendix 4)

The image processing apparatus according to Appendix 3, in which the processor is further configured to deform the solid mesh model in accordance with a boundary condition that is set for the solid mesh model.

### (Appendix 5)

The image processing apparatus according to Appendix 4, in which the processor is further configured to issue a warning when the boundary condition is abnormal.

### (Appendix 6)

The image processing apparatus according to any one of Appendices 1 to 5, in which the three-dimensional organ image is a three-dimensional image of a liver.

### (Appendix 7)

The image processing apparatus according to Appendix 1, in which the processor is further configured to:
acquire a plurality of the three-dimensional organ images in different time phases;
align three-dimensional organ images in two time phases among the plurality of three-dimensional organ images to derive a displacement field representing displacement due to an elapse of time in respective pixels in the three-dimensional organ images in the two time phases; and
derive, in an intermediate time phase between the two time phases, the correspondence points on at least one of the three-dimensional organ images in the two time phases, the correspondence points respectively corresponding to sampling points when volume rendering of a deformed three-dimensional organ image to which the at least one of the three-dimensional organ images in the two time phases is deformed is to be performed, based on the displacement field.

### (Appendix 8)

The image processing apparatus according to Appendix 7, in which the plurality of three-dimensional organ images are three-dimensional organ images of a heart.

### (Appendix 9)

An image processing method including causing a computer to:
acquire a three-dimensional organ image;
derive correspondence points on the three-dimensional organ image, the correspondence points respectively corresponding to sampling points when volume rendering of a deformed three-dimensional organ image to which the three-dimensional organ image is deformed is to be performed; and
use pixel values at the correspondence points on the three-dimensional organ image as pixel values at the sampling points to derive a rendering image which is to be acquired by performing the volume rendering of the deformed three-dimensional organ image.

### (Appendix 10)

An image processing program causing a computer to execute a process including:
acquiring a three-dimensional organ image;
deriving correspondence points on the three-dimensional organ image, the correspondence points respectively corresponding to sampling points when volume rendering of a deformed three-dimensional organ image to which the three-dimensional organ image is deformed is to be performed; and
using pixel values at the correspondence points on the three-dimensional organ image as pixel values at the sampling points to derive a rendering image which is to be acquired by performing the volume rendering of the deformed three-dimensional organ image.

### Reference Signs List

1 computer
2 imaging device
3 image storage server
4 network
11 CPU
12 image processing program
13 storage
14 display
15 input device
16 memory
17 network I/F
18 bus
20 image processing apparatus
21 image acquisition unit
22 extraction unit
23, 23A derivation unit
24 rendering unit
25 display control unit
31 model derivation unit
32 model deformation unit
33 correspondence-point derivation unit
34 text
35 alignment unit
36 correspondence-point derivation unit
41 projection surface
42 viewpoint
43 pixel
44, 47, 49, 52, 71 light ray
45, 48 sampling point
50 point
53, 54 solid mesh
60 display screen
61 first display region
62 second display region
70 displacement field
70A, 70B displacement vector
C1 to C5, C11, C12, C31, C32, C41, C42 correspondence point
GH1, GH2 three-dimensional organ image
O11 to 014, 021 to O24 vertex of solid mesh
RH1 to RH5 rendering image
S1 solid mesh model before deformation
S2 solid mesh model after deformation
SP1 to SP5, SP11, SP12, SP31, SP32 sampling point
V1, V2 three-dimensional image

## Claims

1. An image processing apparatus comprising at least one processor configured to:
acquire a three-dimensional organ image;
derive correspondence points on the three-dimensional organ image, the correspondence points respectively corresponding to sampling points when volume rendering of a deformed three-dimensional organ image to which the three-dimensional organ image is deformed is to be performed; and
use pixel values at the correspondence points on the three-dimensional organ image as pixel values at the sampling points to derive a rendering image which is to be acquired by performing the volume rendering of the deformed three-dimensional organ image.

2. The image processing apparatus according to claim 1, wherein the processor is further configured to:
derive a solid mesh model representing the three-dimensional organ image;
deform the solid mesh model to derive a deformed solid mesh model corresponding to the deformed three-dimensional organ image; and
derive the correspondence points respectively corresponding to the sampling points based on displacement in the deformed solid mesh model from the solid mesh model before deformation.

3. The image processing apparatus according to claim 2, wherein the processor is further configured to:
display the solid mesh model; and
accept deformation of the solid mesh model that is displayed to deform the solid mesh model.

4. The image processing apparatus according to claim 3, wherein the processor is further configured to deform the solid mesh model in accordance with a boundary condition that is set for the solid mesh model.

5. The image processing apparatus according to claim 4, wherein the processor is further configured to issue a warning when the boundary condition is abnormal.

6. The image processing apparatus according to any one of claims 1 to 5, wherein the three-dimensional organ image is a three-dimensional image of a liver.

7. The image processing apparatus according to claim 1, wherein the processor is further configured to:
acquire a plurality of the three-dimensional organ images in different time phases;
align three-dimensional organ images in two time phases among the plurality of three-dimensional organ images to derive a displacement field representing displacement due to an elapse of time in respective pixels in the three-dimensional organ images in the two time phases; and
derive, in an intermediate time phase between the two time phases, the correspondence points on at least one of the three-dimensional organ images in the two time phases, the correspondence points respectively corresponding to sampling points when volume rendering of a deformed three-dimensional organ image to which the at least one of the three-dimensional organ images in the two time phases is deformed is to be performed, based on the displacement field.

8. The image processing apparatus according to claim 7, wherein the plurality of three-dimensional organ images are three-dimensional organ images of a heart.

9. An image processing method comprising causing a computer to:
acquire a three-dimensional organ image;
derive correspondence points on the three-dimensional organ image, the correspondence points respectively corresponding to sampling points when volume rendering of a deformed three-dimensional organ image to which the three-dimensional organ image is deformed is to be performed; and
use pixel values at the correspondence points on the three-dimensional organ image as pixel values at the sampling points to derive a rendering image which is to be acquired by performing the volume rendering of the deformed three-dimensional organ image.

10. An image processing program causing a computer to execute a process comprising:
acquiring a three-dimensional organ image;
deriving correspondence points on the three-dimensional organ image, the correspondence points respectively corresponding to sampling points when volume rendering of a deformed three-dimensional organ image to which the three-dimensional organ image is deformed is to be performed; and
using pixel values at the correspondence points on the three-dimensional organ image as pixel values at the sampling points to derive a rendering image which is to be acquired by performing the volume rendering of the deformed three-dimensional organ image.
